# EUROPEAN PATENT APPLICATION

(11) **EP 4 060 066 A1**
(43) Date of publication of application: **21.09.2022**
(21) Application number: 20887288.7
(22) Date of filing: 12.11.2020
(51) Int. Cl.: C22C 14/00, C22F 1/18

(54) **MEDICAL TITANIUM ALLOY HAVING HIGH FATIGUE STRENGTH, AND HOT PROCESSING AND HOT TREATMENT METHOD THEREFOR AND DEVICE THEREOF**

(30) Priority: 15.11.2019 CN 201911122249
(71) Applicant: Suzhou Silvan Medical Device Co., Ltd., Jiangsu 215000 (CN)
(72) Inventor: WEI, Xiang, Suzhou Industrial Park Jiangsu 215000 (CN); YU, Yachuan, Suzhou Industrial Park Jiangsu 215000 (CN)
(74) Representative: Casalonga
(86) International application number: PCT/CN2020/128354
(87) International publication number: WO 2021/093805

(57) **Abstract**

Disclosed in the present application are a medical titanium alloy having high fatigue strength, and a hot processing and hot treatment method therefor and a device thereof. The medical titanium alloy contains 3.0-6.0% of vanadium, 5.0-7.0% of aluminum, and 4.0-8.0% of copper. By using the described means, the present application enables the medical titanium alloy to have higher fatigue strength.

## Description

### BACKGROUND OF THE PRESENT INVENTION

### FIELD OF INVENTION

The present invention relates to the field of medical materials, and more particularly to a high fatigue strength medical titanium alloy, its thermal processing and heat treatment methods as well as related devices.

### DESCRIPTION OF RELATED ARTS

Since titanium alloy generally has good comprehensive mechanical properties, corrosion resistance, processability and biocompatibility, it is commonly used as medical materials for manufacturing artificial joints, bone nails, bone pins, bone plates, spinal orthopedic internal fixation systems, dental implants, and other important implanted medical devices. It is one of the most important raw materials for implanted or interventional medical devices such as orthopedics, dentistry, and brain surgery.

Accordingly, taking three meals a day as an example, adults normally chew up to 500,000 to 1 million times a year, wherein the tooth bite force usually varies within a range of 2 to 40kg. Under an action of alternating loads, fatigue fracture of implanted artificial dental implants may occur under such conditions below their yield strength. Reports indicated that there were several examples that Ti-6AI-4V alloy dental implants developed in the early stage fractured due to insufficient fatigue strength within 6 months after surgery. With the development of material preparation and processing technology, the performance of titanium alloy is more stable, and the existing short-term success rate of dental implants can reach 98%. However, 15 years of follow-up of patients after implantation found that the implant restoration due to implant failure still exceeded 20%. It has also been reported that adults move one of their hip joints as high as 1 to 2 million times per year. In a normal walking cycle, the hip, knee and ankle joints of a person will bear up to 3 to 10 times the load of their body weight. In other words, the implanted artificial joint materials will bear enormous stress. Arm portions of titanium alloy artificial joint may fracture or break due to insufficient fatigue strength, resulting in serious medical accidents. Statistics has shown that more than 90% of titanium alloy artificial hip joint prostheses require a second restoring operation within 8 to 10 years after surgery, such that it is a huge physical and mental pressure and burden to patients. Therefore, in order to fulfill the effectiveness and long-term safety of clinical treatment for patients, titanium alloy materials are required to have excellent fatigue properties.

It is known that the fatigue strength of metal materials can be enhanced by improving the surface finish of the material and treating the material surface. The smoother the surface (i.e., the lower the roughness), the higher the fatigue strength of the material. However, for medical titanium alloys, in order to enhance the bone bonding ability, it is often necessary to increase its surface roughness for implantation in bones. In other words, it is infeasible to improve the fatigue strength by reducing the surface roughness. Even though the surface treatment of materials, such as surface shot peening, surface nitriding, surface coating, etc., can significantly improve the fatigue strength of the material in a short period of time, the coating will be worn off peeled for the extended service time. It will not only reduce the fatigue strength of the material but also cause a series of biosafety problems. In summary, for the insufficient fatigue strength of medical titanium alloys in clinical applications, it is expected to continuously improve and optimize the existing titanium alloy materials, and develop new titanium alloy with higher fatigue strength.

### SUMMARY OF THE PRESENT INVENTION

The present application solves the technical problem mainly by providing a medical titanium alloy and a hot processing and heat treatment method and related device thereof, which is able to enhance the fatigue strength of the medical titanium alloy.

In order to solve the above technical problems, a technical solution employed by the present application is: a medical titanium alloy, wherein the medical titanium alloy is composed of 3.0-6.0% of vanadium, 5.0-7.0% of aluminum and 4.0-8.0% of copper by weight.

Wherein, the medical titanium alloy contains nanoscale Ti₂Cu phase.

In order to solve the above technical problems, another technical solution employed by the present application is: a method of hot processing of a medical titanium alloy, comprising the steps of: providing a medical titanium alloy ingot, the medical titanium alloy ingot composed of 3.0-6.0% of vanadium, 5.0-7.0% of aluminum and 4.0-8.0% of copper by weight; pre-forging the medical titanium alloy ingot to obtain a medical titanium alloy blank; and forging the medical titanium alloy blank in a multidirectional manner after the medical titanium alloy blank is kept at a temperature of 820-860°C so as to obtain a medical titanium alloy forging piece.

Wherein, the pre-forging step further comprises the steps of: homogenizing said medical titanium alloy ingot for 2 to 4 hours at 950 to 1100°C; and forging the medical titanium alloy ingot in a multiple pass manner, i.e., multi-pass forging process, to obtain the medical titanium alloy blank, wherein the finish forging temperature in the pre-forging step is not lower than 900°C.

Wherein, the step of forging the medical titanium alloy blank in a multidirectional manner after the medical titanium alloy blank is kept at a temperature of 820-860°C comprises the steps of: maintaining the medical titanium alloy blank at 820-860°C for 1 to 3 hours; and forging the medical titanium alloy blank in a multidirectional manner (i.e., multi-directional forging process), to obtain the medical titanium alloy forging piece.

Wherein, the step of providing the medical titanium alloy ingot further comprises the steps of: providing a raw material of the medical titanium alloy, wherein the raw material of said medical titanium alloy is composed of 3.0-6.0% of vanadium, 5.0-7.0% of aluminum and 4.0-8.0% of copper by weight; and smelting and pouring the raw material of the medical titanium alloy to obtain the medical titanium alloy ingot.

Wherein, the step of smelting and pouring the raw material of the medical titanium alloy further comprises a step of: adding the raw material of the medical titanium alloy into an electric arc melting furnace for smelting; or adding the raw material of the medical titanium alloy into a vacuum consumable arc furnace for remelted refining.

Wherein, at least three times of remelted refining are conducted to obtain the medical titanium alloy ingot.

In order to solve the above technical problems, another technical solution employed by the present application is: a method for heat treatment of a medical titanium alloy, which comprises the steps of: providing a medical titanium alloy forging piece, wherein the medical titanium alloy forging piece is composed of 3.0-6.0% of vanadium, 5.0-7.0% of aluminum and 4.0-8.0% of copper by weight; and annealing the medical titanium alloy forging piece to obtain an annealed medical titanium alloy, wherein the medical titanium alloy forging piece is annealed at a temperature of 680-760°C for 0.5-2 hours.

Wherein, after the step of annealing the medical titanium alloy forging piece, the method further comprises a step of: air-cooling the annealed medical titanium alloy to room temperature.

Wherein, before the step of annealing the medical titanium alloy forging piece, the method further comprises a step of: water quenching on the medical titanium alloy forging piece.

Wherein, then the step of providing the medical titanium alloy forging piece comprises: using the above mentioned hot processing method on the medical titanium alloy ingot to obtain the medical titanium alloy forging piece.

In order to solve the above technical problems, another technical solution employed by the present application is: a medical titanium alloy device, wherein the medical titanium alloy device is made of the above mentioned medical titanium alloy.

The advantages of the present invention are: comparing to the prior art, the medical titanium alloy of the present invention is able to improve the overall mechanical properties of the titanium alloy by adding an appropriate amount of copper into the existing titanium alloy, especially, is able to greatly enhance the fatigue strength of titanium alloy.

### BRIEF DESCRIPTION OF THE DRAWINGS

FIG. 1 is an optical microstructure photo of a medical titanium alloy according to a preferred embodiment of the present invention.
FIG. 2 is a transmission microscope microstructure photo of the medical titanium alloy according to the preferred embodiment of the present invention.
FIG. 3 is the X-ray diffraction patterns of the medical titanium alloy according to the preferred embodiment of the present invention.
FIG. 4 is a scanning electron microscope microstructure photo of the medical titanium alloy according to the preferred embodiment of the present invention.
FIG. 5 is an enlarged view of the white rectangular frame in FIG. 4.
FIG. 6 is a scanning electron microscope microstructure photo of the existing medical titanium alloy.
FIG. 7 is a schematic diagram illustrating the dimensions of the fatigue test specimen for the fatigue performance test according to the preferred embodiment of the present invention.

### DETAILED DESCRIPTION OF THE PREFERRED EMBODIMENT

These and other objectives, features, and advantages of the present invention will become apparent from the following detailed description and the accompanying drawings.

In order to develop a new titanium alloy with higher fatigue strength, the present application is arranged to optimize the composition and/or microstructure of the material for enhancing the fatigue strength of the metal material. When the strength of the material is increased, its fatigue strength generally increases. Therefore, in view of materials science, the fatigue properties of medical titanium alloys can be improved by optimizing the composition and/or microstructure of the material.

The present invention provides a medical titanium alloy comprising 3.0-6.0% of vanadium (V), 5.0-7.0% of aluminum (Al) and 4.0-8.0% of copper (Cu) by weight. Preferably, the content of copper can be 4.0%, 4.4%, 4.8%, 5.0%, 5.2%, 5.5%, 5.8%, 6.0%, 6.5%, 7.0%, etc. The content of vanadium can be 3.0%, 4.2%, 5.3%, 6.0%, etc. The content of aluminum can be 5.0%, 5.7%, 6.3%, 7.0%, etc.

Furthermore, the medical titanium alloy is composed of 3.0~6.0% of vanadium, 5.0~7.0% of aluminum and 4.4~5.5% of copper by weight, and the remaining materials are titanium (Ti) and inevitable impurity elements. The content of impurity elements in the alloy should meet the corresponding requirements in the national (Chinese) standard for medical titanium alloys.

According to this preferred embodiment, the mechanical properties of the titanium alloy can be improved by adding an appropriate amount of copper (Cu) element into the titanium alloy. Particularly, the fatigue properties of the titanium alloy can also be improved as well. In other words, an appropriate amount of copper (Cu) is added into the medical Ti-6A1-4V alloy to enhance its fatigue strength.

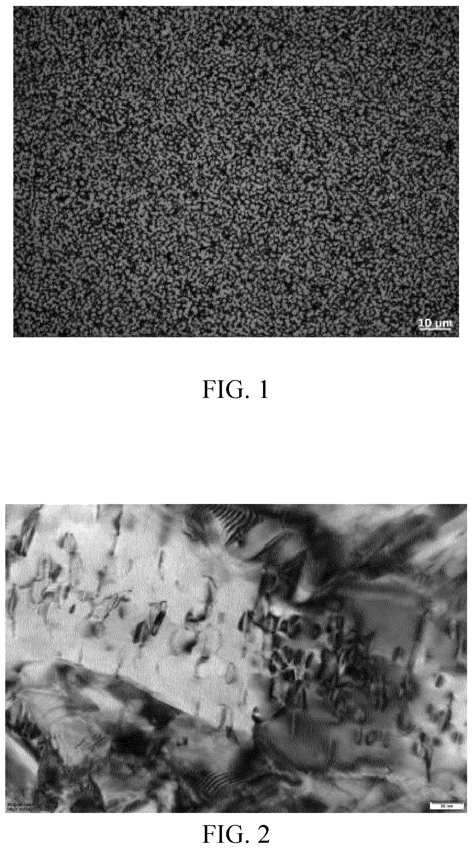

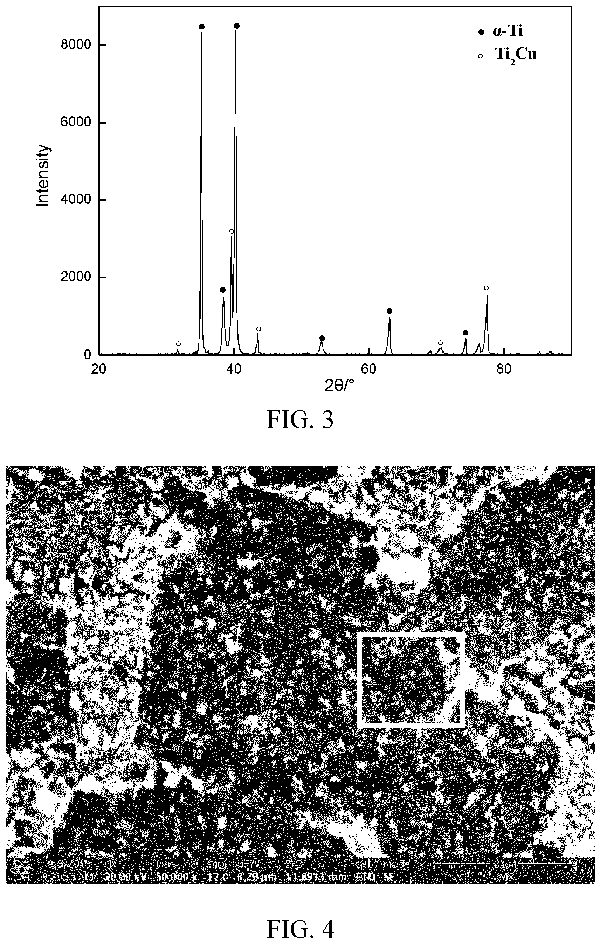

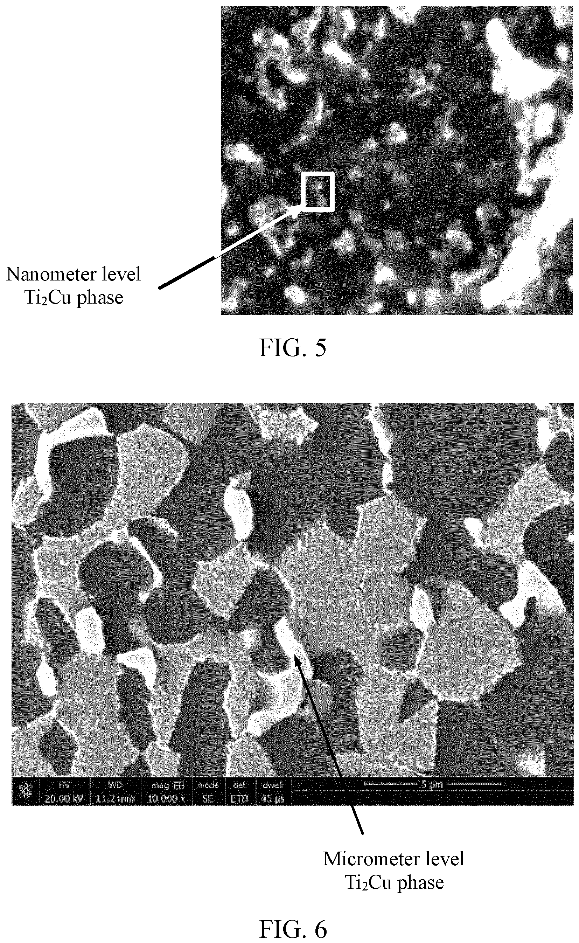

Please refer to FIGs. 1 to 5. FIG. 1 is an optical microstructure photo of a medical titanium alloy according to a preferred embodiment of the present invention, wherein the medical titanium alloy of the present invention has a fully equiaxed microstructure with grain sizes less than 1 µm. FIG. 2 is a transmission microscope microstructure photo of the medical titanium alloy according to the preferred embodiment of the present invention. According to the preferred embodiment, there are nanoscale Ti₂Cu phases in the medical titanium alloy of the present invention. The X-ray diffraction patterns of the medical titanium alloy as shown in FIG. 3 further prove the formation of nanoscale Ti₂Cu phases in the medical titanium alloy of the present invention. FIG. 4 illustrates the morphology of the nano-Ti₂Cu phase under a scanning electron microscope (SEM). FIG. 5 is an enlarged view of a particular area within the white frame in the microstructure photo of the medical titanium alloy in FIG. 4. According to the preferred embodiment of the present invention, the nano-Ti₂Cu phases dispersed and precipitated in the matrix can prevent the dislocation movement in the material during the plastic deformation process, such that the fatigue strength of the titanium alloy material can be greatly enhanced without reducing the ductility of the material.

Referring to Fig. 6, Fig. 6 illustrates a scanning electron microscope microstructure photo of the existing medical titanium alloy. In the existing medical titanium alloy, Ti₂Cu precipitates can be found and the size of the Ti₂Cu precipitates is relatively large (micron scale). The purpose of using such large-sized Ti₂Cu precipitates is to provide a contact sterilization effect so as to improve the antibacterial properties of the material. Comparing the existing medical titanium alloy with the medical titanium alloy of the present invention, it can be found that the two different titanium alloys have different size scales of the Ti₂Cu precipitates and different functions. The existing medical titanium alloy mainly uses the large-sized Ti₂Cu precipitates to provide the contact sterilization effect so as to improve the antibacterial properties of materials. The medical titanium alloy of the present invention mainly uses the dispersed nano-Ti₂Cu precipitates for pinning dislocations so as to enhance the fatigue properties of materials.

The present invention further provides a method for preparing the medical titanium alloy, which comprises three major steps: smelting, hot processing, and heat treatment. Through the hot processing and heat treatment steps, nano-Ti₂Cu phases can be dispersedly precipitated in the medical titanium alloy with addition of copper element.

The smelting step of the method comprises the following steps:

Provide raw material of medical titanium alloy, wherein the raw material of medical titanium alloy contains 3.0~6.0% vanadium, 5.0~7.0% aluminum, 4.0~8.0% copper, and the remaining material of titanium and inevitable impurities.

Smelt and pour the raw material of medical titanium alloy to obtain a medical titanium alloy ingot.

In one example, the raw material of medical titanium alloy can be added into an electric arc melting furnace, wherein the raw material of medical titanium alloy is smelted under predetermined conditions, and is poured to obtain the medical titanium alloy ingot. The specific smelting conditions can be adjustably set for different requirements.

In another example, the raw material of medical titanium alloy can also be added into a vacuum consumable arc melting furnace for remelted refining, wherein at least three remelted refining can be conducted to ensure the uniform distribution of alloying elements.

Subsequently, the medical titanium alloy ingot from the smelting step can be hot processed, and the hot processing step comprises the following steps:

Provide medical titanium alloy ingot, wherein the medical titanium alloy ingot is composed of 3.0~6.0% of vanadium, 5.0~7.0% of aluminum and 4.0~8.0% of copper by weight. The medical titanium alloy ingot can be prepared by the above-mentioned smelting step or by other processes, which should not be limited here.

Pre-forging the medical titanium alloy ingot to obtain a medical titanium alloy blank.

In one example, the medical titanium alloy ingot is homogenized at a temperature of 950-1100°C for 2-4 hours, and then be processed for forging. The medical titanium alloy ingot is forged in a multiple pass manner to obtain the medical titanium alloy blank, wherein the finish forging temperature in the pre-forging step should not be lower than 900°C.

The medical titanium alloy blank is kept at 820-860°C for forging in a multidirectional manner to obtain a medical titanium alloy forging piece.

In one example, the medical titanium alloy blank is maintained at 820-860°C for 1 to 3 hours, and is then forged in a multidirectional manner to obtain the medical titanium alloy forging piece.

Wherein, 820-860°C is the (α+β) dual-phase temperature zone of the titanium alloy. At the temperature of 820-860°C, multidirectional forging processes can be performed to fully break down the internal net-like structure of the material so as to obtain an equiaxed (α+β) structure with a grain size of 3-5 µm.

Then, the medical titanium alloy forging piece can be subsequently performed by a heat treatment process. The heat treatment step is mainly performed by a step of annealing treatment to the medical titanium alloy forging piece, wherein the annealing treatment is performed at a temperature of 680-760°C for 0.5-2 hours. During the annealing treatment step at 680-760°C, the supersaturated Cu element in the material will spontaneously dissolve and precipitate from the α phase to form the nanoscale Ti₂Cu phases. The nano-scale Ti₂Cu phases can strongly hinder the dislocation movement under the cyclic loading so as to significantly enhance the fatigue strength of the material.

In one embodiment, the medical titanium alloy forging piece should be water quenched before the annealing treatment step of the medical titanium alloy forging piece, i.e., immediately after the forging process is completed, to prevent the coarse Ti₂Cu phase precipitation from the β phase during the slow cooling process. Particularly, it is appreciated that through the phase diagram, Cu element has high solid solubility in β phase, but much low solid solubility in α phase, such that the water quenching step being immediately preformed after forging can prevent coarse Ti₂Cu phase precipitation from the β phase.

In one embodiment, after the annealing treatment, the medical titanium alloy annealing member is air-cooled to room temperature to obtain the medical titanium alloy.

According to the above mentioned method, the medical titanium alloys with high fatigue strength can be obtained by adding an appropriate amount of Cu element into the existing medical Ti-6Al-4V alloy, and performing the hot processing and heat treatment for the dispersed precipitation of nano-Ti₂Cu phases. Comparing to the conventional Ti-6Al-4V alloy which is widely used in clinic, the medical titanium alloy of the present invention is strengthened by the nano-Ti₂Cu precipitation to highly enhance the fatigue strength of the medical titanium alloy of the present invention, such that the medical titanium alloy of the present invention is able to provide different applications of medical titanium alloy in medical devices.

According to the above mentioned method, the medical titanium alloy with high fatigue strength can be used for manufacturing different implanted devices. For example, the medical titanium alloys of the present invention can be widely used for various medical devices in various clinical fields such as orthopedics, stomatology and brain surgery to ensure the effectiveness and long-term safety of clinical treatment for patients.

The present invention will be described and explained below through several specific experimental examples and comparative experimental examples, but it should not be limited the scope of the present invention.

The raw materials of titanium alloy in the examples and the comparative examples are prepared respectively, and the specific compositions and proportions of the raw materials are shown in Table 1.

Wherein, in the examples 1 to 4, the smelting process is controlled within the scope of chemical compositions, wherein the content of Cu is gradually increased. In the examples 5 to 7, the smelting process is controlled within the scope of chemical compositions, wherein the chemical compositions are close to each other. The comparative example 1 is a medical Ti-6Al-4V alloy. The comparative example 2 is a medical titanium alloy containing a small amount of Ti₂Cu precipitates. The comparative example 3 is a medical titanium alloy containing a large amount of Ti₂Cu precipitates. The comparative examples 4 to 6 have chemical compositions similar to those of the embodiments 5 to 7.

The raw materials of titanium alloy is smelted to obtain the titanium alloy ingot, wherein the titanium alloy ingot is then subjected to hot processing and heat treatment. The specific processing conditions and parameters are shown in Table 1.

Wherein, the examples 1 to 4 and the comparative examples 1-3 are performed under the same hot processing and heat treatment. Particularly, the pre-forging is performed after the homogenization treatment at 1000°C for 4 hours, and then a multidirectional forging process is performed in the dual-phase region at 820°C. Finally, it is annealed at 740°C for 1 hour, and is then air-cooled to room temperature. By comparing the examples 1 to 4 and the comparative examples 1 to 3, the effect of the amount of nano-Ti₂Cᵤ precipitates on the fatigue properties of the material is illustrated.

The examples 5 to 7 are performed under different heat treatments after the same hot processing, i.e., at different annealing temperatures of 680°C, 720°C and 760°C for 1 hour respectively. The comparative examples 4 to 6 are performed under different hot processing and different heat treatments. The precise forging temperature in the comparative example 4 is higher than the maximum temperature preset in the present invention. The annealing temperature in the comparative example 5 is lower than the lower limit of the annealing temperature preset in the present invention. The annealing temperature in the comparative example 6 is higher than the upper limit of the annealing temperature preset in the present invention. By comparing the comparative examples 4 to 6 and the examples 5 to 7, the effect of different hot processing and heat treatment on the fatigue properties of materials is illustrated.

**Table 1: Chemical compositions, hot processing and heat treatment parameters of medical titanium alloys materials.**

| Serial number | Chemical composition/wt. % | | | | Hot processing | Heat treatment |
|---|---|---|---|---|---|---|
| | Al | V | Cu | Ti | | |
| Example 1 | 5.8 | 4.4 | 4.2 | in balance | 1000°C pre-forging, 820°C precise forging | 740°C annealing 1h |
| Example 2 | 5.9 | 4.3 | 5.3 | in balance | 1000°C pre-forging, 820°C precise forging | 740°C annealing 1h |
| Example 3 | 6.1 | 4.4 | 6.2 | in balance | 1000°C pre-forging, 820°C precise forging | 740°C annealing 1h |
| Example 4 | 6.0 | 4.2 | 7.1 | in balance | 1000°C pre-forging, 820°C precise forging | 740°C annealing 1h |
| Example 5 | 5.9 | 4.3 | 5.5 | in balance | 1000°C pre-forging, 820°C precise forging | 680°C annealing 1h |
| Example 6 | 6.1 | 4.4 | 5.6 | in balance | 1000°C pre-forging, 820°C precise forging | 720°C annealing 1h |
| Example 7 | 6.0 | 4.2 | 5.8 | in balance | 1000°C pre-forging, 820°C precise forging | 760°C annealing 1h |
| Comparative Example 1 | 6.1 | 4.1 | - | in balance | 1000°C pre-forging, 820°C precise forging | 740°C annealing 1h |
| Comparative Example 2 | 5.9 | 4.2 | 0.8 | in balance | 1000°C pre-forging, 820°C precise forging | 740°C annealing 1h |
| Comparative Example 3 | 6.2 | 3.9 | 10.2 | in balance | 1000°C pre-forging, 820°C precise forging | 740°C annealing 1h |
| Comparative Example 4 | 6.2 | 3.9 | 5.7 | in balance | 1000°C pre-forging, 950°C precise forging | 720°C annealing 1h |
| Comparative Example 5 | 5.8 | 4.2 | 5.9 | in balance | 1000°C pre-forging, 820°C precise forging | 400°C annealing 1h |
| Comparative Example 6 | 5.9 | 4.3 | 5.8 | in balance | 1000°C pre-forging, 820°C precise forging | 800°C annealing 1h |

Various performance tests are conducted for the medical titanium alloy materials, and the test methods and standards are as follows:

### 1. Hardness test

The hardness of the titanium alloy material in the examples and the comparative examples are evaluated. The Vickers hardness of each sample of the titanium alloy material after annealing is measured by HTV-1000 hardness tester. The sample surface is polished before testing. The samples are small sheets with 10mm in diameter and 2mm in thickness. The test loading force is 9.8N, and the pressing duration is 15s. By measuring the diagonal length of the indentation mark on the sample, the hardness value is automatically calculated by a hardness analysis software. The final hardness value is the average of 15 points, and three parallel samples are used for each group of samples. The test results are shown in Table 2.

### 2. Tensile test

The tensile mechanical properties of the titanium alloys at room temperature for comparative examples and examples are evaluated by an Instron 8872 tensile testing machine, wherein the tensile rate is 0.5 mm/min. Before the test, the material is machined into a standard tensile sample with a thread diameter of 10 mm, a gauge diameter of 5 mm, and a gauge length of 30 mm. Three parallel samples are taken from each group of heat-treated samples. The mechanical properties obtained from the experiments included tensile strength (σ_{b}), yield strength (σ_{0.2}), elongation (δ) and reduction (Ψ) in area. The test results are shown in Table 2.

### 3. Fatigue test

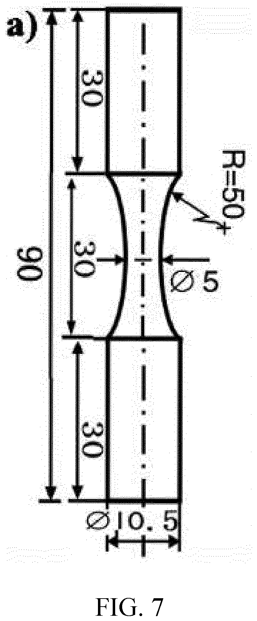

According to the national standard GB 15248-94, the size of the fatigue test sample is shown in Fig. 7. High-cycle fatigue experiments are performed on the titanium alloy materials in the examples and comparative examples by a fatigue testing machine (8800 MiniTower, Instron). The stress loading method is uniaxial tension-tension fatigue, the stress ratio is R=0.1, the frequency is 40 Hz, and the waveform is a *Sine* wave. Starting from a loading 20-30 MPa lower than the tensile strength of the sample, the fatigue life is measured in a descending order by 30-60 MPa each time. Two samples are measured under each stress, and the S-N curve is drawn according to the fatigue life results. The fatigue limit of the material is extrapolated from the S-N curve. The test results are shown in Table 3.

**Table 2: Mechanical properties test results of medical titanium alloy materials.**

| Serial number | Mechanical properties | | | | |
|---|---|---|---|---|---|
| | σ_{b}/MPa | σ_{0.2}/MPa | δ/% | Ψ/% | HV30 |
| Example 1 | 1099 | 1065 | 16.5 | 48 | 325 |
| Example 2 | 1123 | 1083 | 15.0 | 44 | 338 |
| Example 3 | 1153 | 1099 | 13.5 | 38 | 349 |
| Example 4 | 1197 | 1148 | 10.5 | 30 | 361 |
| Example 5 | 1134 | 1095 | 14.0 | 40 | 343 |
| Example 6 | 1105 | 1084 | 15.5 | 46 | 334 |
| Example 7 | 1083 | 1026 | 16.5 | 48 | 324 |
| Comparative Example 1 | 924 | 863 | 17.5 | 54 | 275 |
| Comparative Example2 | 935 | 878 | 17.0 | 50 | 278 |
| Comparative Example 3 | 1285 | 1238 | 3.5 | 14 | 418 |
| Comparative Example 4 | 1301 | 1290 | 3.0 | 19 | 411 |
| Comparative Example 5 | 962 | 858 | 15.0 | 45 | 294 |
| Comparative Example 6 | 974 | 550 | 20.0 | 54 | 301 |

**Table 3: Fatigue test results of medical titanium alloy materials.**

| Serial number | Tensile strength /MPa | Fatigue property /MPa/cycle times | | | | | | | | 10⁷ Fatigue limit/MPa |
|---|---|---|---|---|---|---|---|---|---|---|
| Example 1 | 1099 | 1060 | 1060 | 1030 | 1030 | 1000 | 1000 | 970 | 970 | 991 |
| | | 24539 | 15432 | 448921 | 219394 | 5.9×10⁶ | 2.9×10⁶ | 8.3×10⁷ | 5.7×10⁷ | |
| Example 2 | 1123 | 1090 | 1090 | 1060 | 1060 | 1030 | 1030 | 1000 | 1000 | 1012 |
| | | 19935 | 14956 | 158457 | 234347 | 1.2×10⁶ | 9.8×10⁵ | 3.3×10⁷ | 5.8×10⁷ | |
| Example 3 | 1153 | 1120 | 1120 | 1090 | 1090 | 1060 | 1060 | 1030 | 1030 | 1052 |
| | | 48953 | 78435 | 105832 | 350483 | 4.5×10⁶ | 7.9×10⁶ | 6.3×10⁷ | 8.4×10⁷ | |
| Example 4 | 1197 | 1170 | 1170 | 1140 | 1140 | 1110 | 1110 | 1080 | 1080 | 1076 |
| | | 11493 | 9934 | 23434 | 34985 | 544348 | 873049 | 7.8×10⁶ | 8.4×10⁶ | |
| Example 5 | 1134 | 1100 | 1100 | 1070 | 1070 | 1040 | 1040 | 1010 | 1010 | 1024 |
| | | 39487 | 24395 | 434269 | 847593 | 6.7×10⁶ | 8.5×10⁶ | 7.9×10⁷ | 4.9×10⁷ | |
| Example 6 | 1105 | 1070 | 1070 | 1040 | 1040 | 1010 | 1010 | 980 | 980 | 1003 |
| | | 30493 | 34938 | 787393 | 249803 | 4.3×10⁶ | 6.3×10⁶ | 4.3×10⁷ | 7.7×10⁷ | |
| Example 7 | 1083 | 1050 | 1050 | 1020 | 1020 | 990 | 990 | 960 | 960 | 982 |
| | | 43437 | 75830 | 649945 | 135535 | 2.3×10⁶ | 7.3×10⁶ | 8.5×10⁷ | 5.2×10⁷ | |
| Comparative example 1 | 924 | 890 | 890 | 860 | 860 | 830 | 830 | 800 | 800 | 824 |
| | | 23492 | 34225 | 773539 | 139847 | 5.2×10⁶ | 9.2×10⁶ | 7.2×10⁷ | 9.9×10⁷ | |
| Comparative example 2 | 935 | 910 | 910 | 880 | 880 | 850 | 850 | 820 | 820 | 829 |
| | | 3492 | 10394 | 343476 | 309423 | 1.1×10⁶ | 949497 | 2.3×10⁷ | 4.7×10⁷ | |
| Comparative example 3 | 1285 | 1260 | 1260 | 1200 | 1200 | 1140 | 1140 | 1080 | 1080 | 962 |
| | | 92 | 293 | 747 | 1781 | 13244 | 34534 | 84987 | 74324 | |
| Comparative example 4 | 1301 | 1270 | 1270 | 1210 | 1210 | 1150 | 1150 | 1090 | 1090 | 961 |
| | | 41 | 98 | 378 | 221 | 9243 | 28902 | 27432 | 12769 | |
| Comparative example 5 | 962 | 930 | 930 | 900 | 900 | 870 | 870 | 840 | 840 | 852 |
| | | 7349 | 18342 | 378492 | 129485 | 3.7×10⁶ | 1.2×10⁶ | 3.1×10⁷ | 2.2×10⁷ | |
| Comparative example 6 | 974 | 930 | 930 | 900 | 900 | 870 | 870 | 840 | 840 | 863 |
| | | 12947 | 67943 | 783942 | 393293 | 2.9×10⁶ | 6.2×10⁶ | 7.9×10⁷ | 8.2×10⁷ | |

Comparing with the medical Ti-6Al-4V alloy in the comparative example (Comp. Eg.) 1, as shown from the results in Table 2, examples (Eg.) 1 to 7 not only have higher hardness but also have good match of strength and plasticity.

The content of copper plays an important role for the strength, hardness, plasticity and fatigue properties of the titanium alloy material. Within the range of Cu content according to the present invention, by increasing the Cu content, both strength and hardness of the material are improved, while the plasticity is slightly reduced (examples 1 to 4). Cu content in the comparative example 2 is lower, wherein its mechanical properties are similar to those of the medical Ti-6Al-4V alloy in the comparative example 1. Cu content in the comparative example 3 is as high as 10.2%, wherein even though the material has a relative higher strength, its elongation and reduction in area are only 3.5% and 14%, respectively, which are far lower than the lower limit in the national (Chinese) standard of "Titanium and Titanium Alloy Processing Materials for Surgical Implants".

The hot processing and heat treatment also play important roles for the microstructure of titanium alloy materials. In the comparative example 4, due to the high forging temperature, the martensitic plate structure is obtained after forging, resulting in high hardness but poor plasticity of the material. Since Ti₂Cu phase is difficult to precipitate in the comparative examples 5 and 6 during annealing, both hardness and strength of the materials are lower.

As shown from the results in Table 3, copper content, hot processing and heat treatment play important roles for the fatigue properties of titanium alloy materials. The fatigue strength of the titanium alloy materials in examples 1 to 4 gradually increased by increasing the Cu content, wherein in the example 4, the Cu content of 7.1% and its fatigue strength is as high as 1076 MPa, the increase is more than 30% when comparing to the fatigue strength 824 MPa of titanium alloy Ti-6Al-4V in the comparative example 1,. Examples 5-7 show that by increasing the annealing temperature, the fatigue strength of the material decreases slightly. This is because when the annealing temperature is increased, the Ti₂Cu phases that strengthen the material are coarsened. The comparative examples 2 and 3 show that when the Cu content in the material is higher or lower than the Cu content as defined in the present invention, the strength of the material is greatly reduced. In the comparative example 4, the forging is not conducted at the temperature as defined in the present invention, wherein the equiaxed structure is not obtained, resulting in a significant decrease in fatigue property. In the comparative examples 5 and 6, the heat treatment is not performed at the temperature as defined in the present invention, and the Ti₂Cu phase could not precipitate during the heat treatment, resulting in lower fatigue strength of the material.

Furthermore, the change of the element content definitely affects the subsequent heat treatment process, wherein the heat treatment determines the properties of the titanium alloy material. Therefore, from the results in the above examples and comparative examples, it is appreciated that only when the content of each element in the titanium alloy material, the hot processing and the heat treatment process are within a certain range, these factors complement and cooperate with each other, so as to possess high fatigue properties, good tensile properties and hardness for the titanium alloy material. For the adjustment of Cu content and heat treatment parameters, the present invention is arranged to provide and establish different experimental conditions in order to analyze the experimental results (for example, if one of the experimental results shows that both hardness and fatigue properties become lower, but the plasticity is improved, it needs to be analyzed whether it is caused by the change of Cu content or the change of heat treatment parameters). For details, referring to experimental phenomena, literature, etc., the direction of subsequent experiments can be determined to verify the analysis and judgment. Then, by adjusting the experimental direction again, a better and suitable experimental plan with a smaller number of experiments can be obtained to determine the material composition and processing parameters.

According to the preferred embodiment, the medical titanium alloy of the present invention can be obtained by adding an appropriate amount of Cu element into the existing medical Ti-6Al-4V alloy, incorporated with the hot processing and heat treatment for the dispersed precipitation of nano-Ti₂Cu phases, so as to enhance the fatigue strength with good strength-plastic matching of the medical titanium alloy. The medical titanium alloy can be widely used in various medical devices in clinics for orthopedics, stomatology, and brain surgery, so as to ensure the effectiveness and long-term safety of clinical treatment for the patients.

The above description is an illustration of the embodiments of the present invention and is not intended to limit the scope of the present invention in the application. Any modification or transformation of equivalent structure or equivalent process based on the description and drawings of the present invention, or any applications directly or indirectly applied in other related technologies fields, are both encompassed within the scope of the present invention in the application.

## Claims

1. A medical titanium alloy, **characterized in that**: the medical titanium alloy is composed of 3.0-6.0% of vanadium, 5.0-7.0% of aluminum and 4.0-8.0% of copper by weight.

2. The medical titanium alloy according to claim 1, **characterized in that**: said medical titanium alloy contains 3.0-6.0% of said vanadium, 5.0-7.0% of said aluminum and 5.0-8.0% of said copper by weight.

3. The medical titanium alloy according to claim 1, **characterized in that**: said medical titanium alloy contains nanoscale Ti₂Cu phases.

4. A method of hot processing of a medical titanium alloy, **characterized in that**:
comprising the steps of:
providing a medical titanium alloy ingot, said medical titanium alloy ingot composed of 3.0-6.0% of vanadium, 5.0-7.0% of aluminum and 4.0-8.0% of copper by weight;
pre-forging said medical titanium alloy ingot to obtain a medical titanium alloy blank; and
forging said medical titanium alloy blank in a multidirectional manner after said medical titanium alloy blank is kept at 820-860°C so as to obtain a medical titanium alloy forging piece.

5. The method of hot processing of a medical titanium alloy according to claim 4, **characterized in that**: said medical titanium alloy ingot contains 3.0-6.0% of vanadium, 5.0-7.0% of aluminum and 5.0-8.0% of copper by weight.

6. The method of hot processing of a medical titanium alloy according to claim 4, **characterized in that**: said pre-forging step further comprises the steps of:
homogenizing said medical titanium alloy ingot for 2 to 4 hours at 950 to 1100°C; and
forging said medical titanium alloy ingot in a multiple pass manner to obtain said medical titanium alloy blank, wherein the finish forging temperature in said pre-forging step is not lower than 900°C.

7. The method of hot processing of a medical titanium alloy according to claim 4, **characterized in that**: said forging step further comprises the steps of:
maintaining said medical titanium alloy blank at 820-860°C for 1 to 3 hours; and
forging said medical titanium alloy blank in a multidirectional manner to obtain said medical titanium alloy forging piece.

8. The method of hot processing of a medical titanium alloy according to claim 4, **characterized in that**: wherein said step of providing said medical titanium alloy ingot further comprises the steps of:
providing a raw material of the medical titanium alloy, wherein said raw material of said medical titanium alloy is composed of 3.0-6.0% of vanadium, 5.0-7.0% of aluminum and 4.0-8.0% of copper by weight; and
smelting and pouring said raw material of said medical titanium alloy to obtain said medical titanium alloy ingot.

9. The method of hot processing of a medical titanium alloy according to claim 8, **characterized in that**: wherein said smelting step further comprises one of the steps of:
adding said raw material of said medical titanium alloy into an electric arc smelting furnace for smelting; or
adding said raw material of said medical titanium alloy into a vacuum consumable arc melting furnace for remelted refining.

10. A method for heat treatment of a medical titanium alloy, **characterized in that**:
comprising the steps of:
providing a medical titanium alloy forging piece, wherein said medical titanium alloy forging piece is composed of 3.0-6.0% of vanadium, 5.0-7.0% of aluminum and 4.0-8.0% of copper by weight; and
annealing said medical titanium alloy forging piece to obtain a medical titanium alloy annealing piece, wherein said medical titanium alloy forging piece is annealed at a temperature of 680-760°C for 0.5-2 hours.

11. The method for heat treatment of a medical titanium alloy according to claim 10, **characterized in that**: wherein said medical titanium alloy forging piece is composed of 3.0-6.0% of vanadium, 5.0-7.0% of aluminum and 5.0-8.0% of copper by weight.

12. The method for heat treatment of a medical titanium alloy according to claim 10, **characterized in that**: after the annealing step, further comprising a step of:
air-cooling said medical titanium alloy annealing piece to room temperature.

13. The method for heat treatment of a medical titanium alloy according to claim 10, **characterized in that**: before the annealing step, further comprising a step of:
water quenching on said medical titanium alloy forging piece.

14. The method for heat treatment of a medical titanium alloy according to claim 10, **characterized in that**: wherein said step of providing said medical titanium alloy ingot further comprises a step of:
performing the method as claimed in any one of claims 4 to 9, wherein said medical titanium alloy ingot is hot processed to obtain said medical titanium alloy forging piece.

15. A medical titanium alloy device, **characterized in that**: said medical titanium alloy device is made of the medical titanium alloy as claimed in any one of claims 1-3.
